# EUROPÄISCHE PATENTSCHRIFT

(11) **EP 2 182 497 B1**
(45) Veröffentlichungstag und Bekanntmachung des Hinweises auf die Patenterteilung: **10.11.2010**
(21) Anmeldenummer: 09010172.6
(22) Anmeldetag: 06.08.2009
(51) Int. Cl.: G08B 17/10, G08B 21/16, G01N 33/00, H05K 1/18

(54) **Gasdetektor**
Gas detector
Détecteur de gaz

(30) Priorität: 30.10.2008 DE 102008053909
(43) Veröffentlichungstag der Anmeldung: 05.05.2010
(73) Patentinhaber: Novar GmbH, 41469 Neuss (DE)
(72) Erfinder: Ollik, Waldemar, 47495 Rheinberg (DE); Politze, Heiner, 41469 Neuss (DE); Krippendorf, Tido, 41812 Erkelenz (DE); Ankara, Zafer, 41466 Neuss (DE)
(74) Vertreter: Prietsch, Reiner

(56) Entgegenhaltungen:
- EP-A1- 1 376 505
- DE-A1- 10 303 263
- DE-A1- 19 810 060
- GB-A- 2 306 218
- US-A1- 2005 285 039
- US-B1- 6 483 324

## Beschreibung

Die Erfindung betrifft einen Brandmelder mit den Merkmalen des Oberbegriffes des Anspruches 1.

Aus der EP 1 376 501 A und der GB 2 306 218 A ist ein Brandmelder dieser Gattung bekannt, bei dem anstelle des im Einzelnen beschriebenen optischen Sensorsystems auch ein Gassensor vorgesehen sein kann. Hierfür sind eine Halterung in der Rauchkammer und eine Verdrahtung des Gassensors mit der Schaltungsplatine erforderlich.

Aus der DE 198 10 060 A ist ein Gassensor mit einem SMD-fähig ausgebildeten Gehäuse bekannt, das auf einer Schaltungsplatine so aufgelötet ist, dass die Gaseintrittsfläche mit einer Öffnung in der Schaltungsplatine fluchtet.

Übliche Gassensoren haben Gehäuse wie z.B. TO 39, die wegen ihrer der Bodenseite mit den Anschlussdrähten gegenüberliegenden Gaseintrittsfläche nicht mittels der üblichen Bestückungsautomaten handhabbar sind und deshalb mit ihren Anschlussdrähten von Hand in die Bohrungen in der im übrigen SMD-bestückten Schaltungsplatine eingesetzt werden müssen. In einigen Fällen, z.B. bei Verwendung des Gasdetektors als Teil eines Streulichtbrandmelders, ist außerdem nachteilig, dass die Gaseintrittsfläche des Gassensors sich auf der Bestückungsseite der Schaltungsplatine befindet, jedoch von dieser abgewandt ist. Die Schaltungsplatine muss dann so positioniert und in dem Gehäuse des Streulichtbrandmelders angeordnet sein, dass nur die Gaseintrittsfläche des Gassensors über eine Öffnung in einer Wand der Rauchkammer des Streulichtbrandmelders mit dieser Rauchkammer im Gasaustausch steht.

Der Erfindung liegt die Aufgabe zugrunde, einen Gasdetektor der einliegend angegebenen Gattung zu schaffen, dessen Schaltungsplatine mittels eines üblichen Bestückungsautomaten mit dem Gassensor bestückbar ist.

Diese Aufgabe ist durch die kennzeichnenden Merkmale des Anspruches 1 gelöst.

Vorzugsweise ist die der Gaseintrittsfläche abgewandte Seite des Gehäuses des Gassensors zur Handhabung mittels eines SMD-Bestückungsautomaten ausgebildet. Dazu hat das Sensorgehäuse auf der der Schaltungsplatine abgewandten Seite eine ebene, ausreichend mechanisch belastbare Fläche, an der ein SMD-Bestückungsautomat, z.B. mit einer Saugpipette, angreifen kann.

Wenn der Gassensor so auf die Öffnung der Schaltungsplatine aufgelötet ist, dass er die Öffnung abdichtet, dann kommuniziert die Gaseintrittsfläche ausschließlich über die Öffnung mit der Umgebung. Die Schaltungsplatine kann dann einen Raum, der auf ein Gas zu überwachen ist, von einem nicht zu überwachenden Raum trennen. Die Abdichtung kann beispielsweise durch eine umlaufende Dichtlippe, die am Gehäuse des Gassensors angebracht ist, erfolgen.

Zum Schutz einer hinter der Gaseintrittsfläche liegenden Gassensorik, z.B. einer Sensorschicht, kann die Gaseintrittsfläche des Gehäuses des Gassensors vorzugsweise mit einer gasdurchlässigen Schutzfolie abgedeckt sein. Bei ausreichender Festigkeit der Schutzfolie ist dann eine Handhabung des Gassensors auch mittels einer auf diese Schutzfolie aufsetzenden Saugpipette möglich.

Sollen nur bestimmte Gase detektiert werden, dann ist es vorteilhaft, wenn in dem Gehäuse des Gassensors hinter der Gaseintrittsfläche ein Gasfilter angeordnet ist. Der Gasfilter kann insbesondere zwischen der Schutzfolie und einer Gassensorik angeordnet sein.

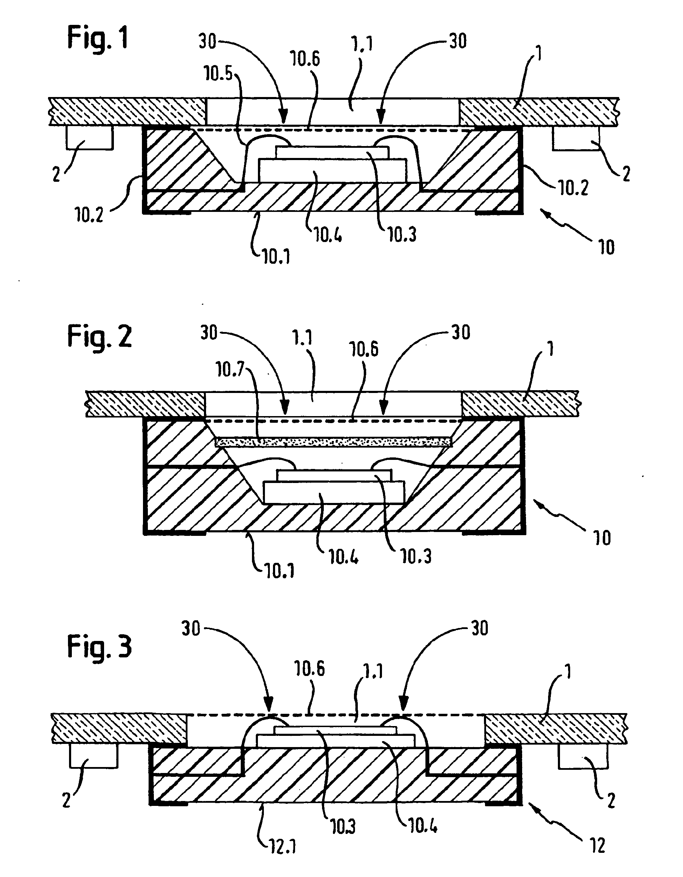

Anhand der Zeichnung wird die Erfindung schematisch vereinfacht und beispielhaft beschrieben. Die Figuren 1 bis 3 zeigen je einen Teil der Schaltungsplatine eines Gasdetektors mit unterschiedlichen Ausführungsformen des Gassensors.

Der Gasdetektor in Figur 1 hat eine Schaltungsplatine 1 mit einer Auswerteschaltung, die durch SMD-Bauteile 2 angedeutet ist.

Auf die Bestückungsseite der Schaltungsplatine 1 ist in Höhe einer Öffnung 1.1 der Schaltungsplatine 1 ein Gassensor 10 aufgelötet, dessen Gehäuse in SMD-Technik ausgebildet ist. Das Gehäuse hat auf seiner der Öffnung 1.1 zugewandten Seite eine Gaseintrittsfläche und auf der gegenüberliegenden Seite eine Bodenfläche 10.1. An seinen Seitenflächen hat das Gehäuse die üblichen Lötpads 10.2, die jedoch über die volle Höhe der Seitenflächen des Gehäuses herumgezogen sind um den Gassensor 10 auch auf Schaltungsplatinen ohne die Öffnung 1.1 und dementsprechend mit von der jeweiligen Bestückungsseite abgewandter Gaseintrittsöffnung auf- bzw. einlöten zu können.

Der innere Aufbau des Gassensors 10 unterscheidet sich nicht prinzipiell von demjenigen eines üblichen Gassensors und ist deshalb mit einer Sensorschicht 10.3 auf einem Träger 10.4 sowie Bonddrähten 10.5 zu den Lötpads 10.2 nur schematisch angedeutet.

Die Gaseintrittsöffnung des Gassensors 10 ist mit einer Schutzfolie 10.6 abgedeckt, die selbstverständlich gasdurchlässig sein muss. Bei ausreichender Festigkeit der Schutzfolie 10.6 kann der Gassensor 10 mittels der Saugpipette eines üblichen Bestückungsautomaten nicht nur vom Boden 10.1 des Gehäuses sondern auch von der Seite der Schutzfolie 10.6 aus gehandhabt werden.

In der Ausführungsform gemäß Fig. 2 ist in die Gaseintrittsfläche des Gassensors 10 ein zusätzliches Gasfilter 10.7 eingesetzt, das bevorzugt nur eine bestimmte Gasart hindurch lässt, so dass der Gassensor 10 für diese Gasart besonders empfindlich ist.

Fig. 3 zeigt einen Gassensor 12 mit frei liegender Sensorschicht 12.3. In diesem Fall ist die Öffnung 1.1 in der Schaltungsplatine 1 durch die gasdurchlässige Schutzfolie 10.1 auf der Gaseintrittseite abgedeckt.

In allen Figuren ist mit den Pfeilen 30 der Gaszutritt angedeutet. Der Einfachheit halber nicht dargestellt sind die gedruckten Leiterbahnen, die Lötpads und die Lotstellen selbst.

## Patentansprüche

1. Brandmelder mit einer Rauchkammer und mit einem Gasdetektor, der eine gedruckte Schaltungsplatine (1) mit einem Gassensor (10) und einer Auswerteschaltung (2) umfasst, **dadurch gekennzeichnet, dass** der Gassensor (10) ein Gehäuse (11) mit einer Gaseintrittsfläche (13) umfasst, dass das Gehäuse (11) des Gassensors (10) SMD-fähig ausgebildet und mit seiner Gaseintrittsfläche (13) mit einer Öffnung (1.1) in der Schaltungsplatine (1) fluchtend auf diese aufgelötet ist und dass die Schaltungsplatine (1) mit ihrer dem Gassensor (10) abgewandten Seite an die Rauchkammer derart angrenzt, dass der Gassensor (10) über die Öffnung (1.1) in der Schaltungsplatine (1) im Gasaustausch mit der Rauchkammer steht.

2. Brandmelder nach Anspruch 1, **dadurch gekennzeichnet, dass** die der Gaseintrittsfläche abgewandte Seite (10.1) des Gehäuses des Gassensors (10) zur Handhabung mittels eines SMD-Bestückungsautomaten ausgebildet ist.

3. Brandmelder nach Anspruch 2, **dadurch gekennzeichnet, dass** die der Gaseintrittsfläche abgewandte Seite (10.1) des Gehäuses des Gassensors (10) zur Handhabung mittels einer Saugpipette ausgebildet ist.

4. Brandmelder nach einem der Ansprüche 1 bis 3, **dadurch gekennzeichnet, dass** der Gassensor (10) die Öffnung (1.1) in der Schaltungsplatine (1) abdichtend aufgelötet ist.

5. Brandmelder nach einem der Ansprüche 1 bis 4, **dadurch gekennzeichnet, dass** die Gaseintrittsfläche des Gehäuses des Gassensors (10) mit einer gasdurchlässigen Schutzfolie (10.6) abgedeckt ist.

6. Brandmelder nach einem der Ansprüche 1 bis 5, **dadurch gekennzeichnet, dass** in dem Gehäuse des Gassensors (10) hinter der Gaseintrittsfläche ein Gasfilter (10.7) angeordnet ist.

## Claims

1. A fire detector, comprising a smoke chamber and a gas detector which comprises a printed circuit board (1) with a gas sensor (10) and an evaluation circuit (2), **characterized in that** the gas sensor (10) comprises a housing (11) with a gas entrance surface (13), the housing (11) of the gas sensor (10) being designed as SMD and is surface-mounted on the printed circuit board (1) with its gas entrance surface (13) in alignment with an opening (1.1) of the same, and the printed circuit board (1) borders on the smoke chamber with its side facing away from the gas sensor (10) such that the gas sensor (10) is in gas exchange with the smoke chamber via the opening (1.1) in the printed circuit board (1).

2. A fire detector according to claim 1, **characterized in that** the side (10.1) of the housing of the gas sensor (10) which faces away from the gas entrance surface is arranged for handling by means of an automatic SMD pick-and-place machine.

3. A fire detector according to claim 2, **characterized in that** the side (10.1) of the housing of the gas sensor (10) opposite the gas entrance surface is arranged for handling by means of a suction pipette.

4. A fire detector according to one of the claims 1 to 3, **characterized in that** the gas sensor (10) is surface-mounted to seal the opening (1.1) in the printed circuit board (1).

5. A fire detector according to one of the claims 1 to 4, **characterized in that** the gas entrance surface of the housing of the gas sensor (10) is covered with a gas-permeable protective foil (10.6).

6. A fire detector according to one of the claims 1 to 5, **characterized in that** a gas filter (10.7) is arranged in the housing of the gas sensor (10) behind the gas entrance surface.

## Revendications

1. Détecteur d'incendie avec une chambre à fumée et avec un détecteur de gaz comprenant une carte de circuits imprimés (1) avec un capteur de gaz (10) et un circuit d'analyse (2), **caractérisé en ce que** le capteur de gaz (10) comprend un boîtier (11) avec une surface d'entrée des gaz (13), **en ce que** le boîtier (11) du capteur de gaz (10) est conçu pour pouvoir être monté en surface et est brasé par sa surface d'entrée des gaz (13) avec une ouverture (1.1) dans la carte de circuits (1) en affleurement sur celle-ci, et **en ce que** la carte de circuits (1) est contiguë de la chambre à fumée sur sa face opposée au capteur de gaz (10) de telle façon que le capteur de gaz (10) est en situation d'échange gazeux avec la chambre à fumée à travers l'ouverture (1.1) de la carte de circuits (1).

2. Détecteur d'incendie selon la revendication 1, **caractérisé en ce que** le côté (10.1) du boîtier du capteur de gaz (10) opposé à la surface d'entrée des gaz est conçu pour pouvoir être manipulé par un appareil automatique d'assemblage de composants montés en surface.

3. Détecteur d'incendie selon la revendication 2, **caractérisé en ce que** le côté (10.1) du boîtier du capteur de gaz (10) opposé à la surface d'entrée des gaz est conçu pour pouvoir être manipulé au moyen d'une pipette d'aspiration.

4. Détecteur d'incendie selon l'une des revendications 1 à 3, **caractérisé en ce que** le capteur de gaz (10) est brasé de façon à fermer l'ouverture (1.1) dans la carte de circuits (1).

5. Détecteur d'incendie selon l'une des revendications 1 à 4, **caractérisé en ce que** la surface d'entrée des gaz du boîtier du capteur de gaz (10) est recouverte d'un film protecteur (10.6) perméable aux gaz.

6. Détecteur d'incendie selon l'une des revendications 1 à 5, **caractérisé en ce qu'**un filtre à gaz (10.7) est disposé dans le boîtier du capteur de gaz (10) derrière la surface d'entrée des gaz.
